(19) Europäisches Patentamt / European Patent Office / Office européen des brevets

(11) EP 3 530 681 A1

(12) EUROPEAN PATENT APPLICATION
published in accordance with Art. 153(4) EPC

(43) Date of publication:
**28.08.2019 Bulletin 2019/35**

(21) Application number: **17861822.9**

(22) Date of filing: **09.08.2017**

(51) Int Cl.:
***C08G 8/30*** (2006.01) ***C07C 39/04*** (2006.01)
***C07C 39/07*** (2006.01)

(86) International application number:
**PCT/JP2017/028997**

(87) International publication number:
**WO 2018/074040 (26.04.2018 Gazette 2018/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **18.10.2016 JP 2016204487**

(71) Applicant: **Showa Denko K.K.**
**Tokyo 105-8518 (JP)**

(72) Inventors:
- **HASEGAWA, Aoi**
  **Tokyo 105-8518 (JP)**
- **ISHIBASHI, Yoshitaka**
  **Tokyo 105-8518 (JP)**

(74) Representative: **Strehl Schübel-Hopf & Partner**
**Maximilianstrasse 54**
**80538 München (DE)**

# (54) METHOD FOR PRODUCING POLYALKENYLPHENOL COMPOUND, CURABLE COMPOSITION INCLUDING POLYALKENYLPHENOL COMPOUND, AND CURED PRODUCT OF CURABLE COMPOSITION

(57) Provided is a method with which it is possible to produce a polyalkenylphenol compound having a narrow molecular weight distribution and low viscosity at high purity and high yield. A poly 2-alkenyl aromatic ether compound having two or more phenol skeletons is subjected to Claisen rearrangement in the presence of a phenol or naphthol compound.

EP 3 530 681 A1

## Description

FIELD

The present invention relates to a method for producing a polyalkenylphenol compound, a curable composition containing a polyalkenylphenol compound, and a cured product thereof.

BACKGROUND

The [3,3]-sigmatropic rearrangement of alkenyl phenyl ethers, which had been discovered by Claisen, et al. in 1912, is called an aromatic Claisen reaction. Using a phenol compound as a raw material, various phenol compounds having an alkenyl group are obtained through an alkenyl etherification using an alkenyl halide, etc., and the subsequent Claisen reaction under heating conditions. The alkenyl group after rearrangement gives a building block in various derivatizations, such as oxidation, metathesis and coupling, and in turn, gives an intermediate useful in synthesis, and the rearrangement above is therefore one of important synthesis methods for industrial products, pharmaceutical and agrochemical products, etc.

Conventionally, the Claisen rearrangement reaction has been carried out using, for example, an alkenyl ether (sometimes containing a byproduct salt) at high temperature of around 200°C in the presence of a high-boiling-point solvent, such as carbitol, paraffin oil and N,N-dimethylaniline, or in the absence of a solvent. The reaction in the presence of a solvent is described, for example, in Patent Document 1 (Japanese Unexamined Patent Publication No. 2004-137200) and Patent Document 2 (Japanese Unexamined Patent Publication No. 2003-104923). In Patent Document 1, a diallyl ether of 4,4'-(9-fluorenylidene)diphenol is Claisen-rearranged at 200°C in the presence of N,N-diethylaniline. In Patent Document 2, a mixture of bisphenol A diallyl ether and sodium chloride is Claisen-rearranged at 190°C in the presence of 4,4'-isopropylidenebis(2,6-di-tert-butylphenol).

[CITATION LIST]

[PATENT LITERATURE]

[PTL 1] Japanese Unexamined Patent Publication No. 2004-137200
[PTL 2] Japanese Unexamined Patent Publication No. 2003-104923

SUMMARY

[TECHNICAL PROBLEM]

In the conventional liquid-phase reaction in the presence of a solvent described in Patent Document 1, it is known that production of phenol due to elimination of an alkenyl group, polymerization by a radical reaction between alkenyl groups, etc., proceeds as a side reaction and the molecular weight of the product increases. An alkenylphenol compound produced by the method above has high viscosity and therefore, moldability, coatability, etc., may be compromised.

In Patent Document 2, since a phenol compound having a high boiling point is used, the phenol compound may possibly remain as an impurity in the system without being removed after the reaction.

In consideration of these circumstances, an object of the present invention is to provide a method for producing a polyalkenylphenol compound from a poly 2-alkenyl aromatic ether compound by using a Claisen rearrangement reaction, in which suppression of a side reaction, such as polymerization by a radical reaction between alkenyl groups, oxidation of a polyalkenylphenol compound, and resulting coloration, may be expected and a polyalkenylphenol compound having a narrow molecular weight distribution and a low viscosity can be obtained in high yield.

[SOLUTION TO PROBLEM]

As a result of intensive studies, the present inventors have found that when an alkenyl group of a poly 2-alkenyl aromatic ether compound having at least two specific structural units is Claisen-rearranged in the presence of an aromatic compound having at least one specific phenolic hydroxyl group, suppression of a side reaction, such as polymerization by a radical reaction between alkenyl groups, can be expected and a polyalkenylphenol compound having a narrow molecular weight distribution and a low viscosity is obtained in high yield. The present invention has been accomplished

based on this finding.

More specifically, the present invention includes the following embodiments.

[1] A method for producing a polyalkenylphenol compound (C), including Claisen-rearranging an alkenyl group of a compound (A) having at least two structural units represented by any of the following formulae (1a), (1b) and (1c):

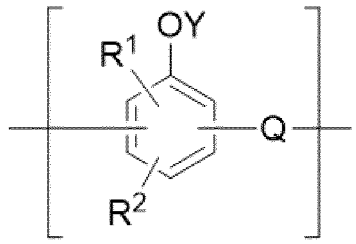

(1a)

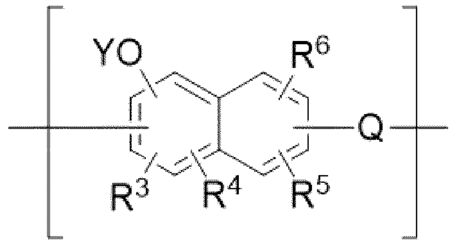

(1b)

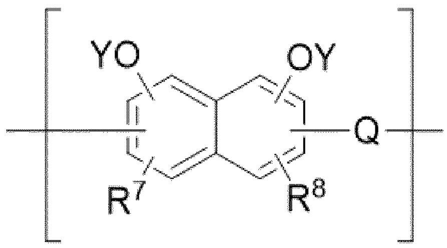

(1c)

wherein, in formulae (1a) to (1c), each of $R^1$ to $R^8$ independently represents a hydrogen atom, an alkyl group having a carbon number of 1 to 10, an alkoxy group having a carbon number of 1 to 2, or a hydroxyl group, $R^3$ to $R^8$ may be located on any carbon atom constituting the naphthalene ring, each Q is independently an alkylene group represented by the formula - $CR^9R^{10}$-, a cycloalkylene group having a carbon number of 5 to 10, a divalent organic group having an aromatic ring, a divalent organic group having an alicyclic fused ring, or a divalent group formed by combination thereof, each of $R^9$ and $R^{10}$ independently represents a hydrogen atom, an alkyl group having a carbon number of 1 to 5, an alkenyl group having a carbon number of 2 to 6, a cycloalkyl group having a carbon number of 5 to 10, or an aryl group having a carbon number of 6 to 12, Y is an alkenyl group represented by the following formula (2):

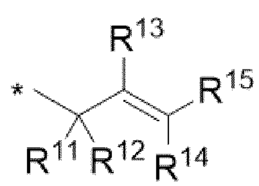

(2)

each of $R^{11}$, $R^{12}$, $R^{13}$, $R^{14}$ and $R^{15}$ independently represents a hydrogen atom, an alkyl group having a carbon number of 1 to 5, a cycloalkyl group having a carbon number of 5 to 10, or an aryl group having a carbon number of 6 to 12, and * in formula (2) represents a bonding site to an oxygen atom,

in the presence of at least one compound (B) represented by either the following formula (3) or (4):

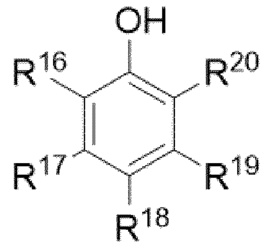

(3)

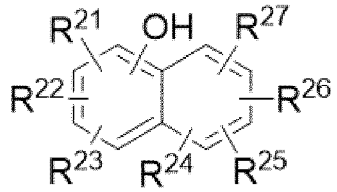

(4)

wherein, in formulae (3) and (4), each of $R^{16}$ to $R^{27}$ independently represents a hydrogen atom, an alkyl group having a carbon number of 1 to 10, a cycloalkyl group having a carbon number of 3 to 12, or an aryl group having a carbon number of 6 to 10.

[2] The method according to [1], wherein the compound (B) is a compound represented by formula (3).

[3] The method according to [1] or [2], wherein the compound (B) is a compound represented by formula (3) and each of $R^{16}$ to $R^{20}$ is independently a hydrogen atom or a methyl group.

[4] The method according to any one of [1] to [3], wherein the compound (B) is at least one compound selected from the group consisting of phenol, o-cresol, m-cresol, and p-cresol.

[5] The method according to any one of [1] to [4], wherein the compound (A) has at least two structural units represented by either formula (1a) or (1b).

[6] The method according to any one of [1] to [5], wherein the compound (A) has at least two structural units represented by formula (1a).

[7] The method according to any one of [1] to [6], wherein in the compound (A), the average per molecule of the total number of structural units represented by any of formulae (1a), (1b) and (1c) is from 2 to 20.

[8] The method according to any one of [1] to [7], wherein the rate of molecular weight increase defined by the following formula is from 0 to 70%:

$$\text{Rate of molecular weight increase [\%]} = (m_C / m_A - 1) \times 100,$$

wherein $m_A$ represents the weight average molecular weight of the compound (A) and $m_C$ represents the weight average molecular weight of the polyalkenylphenol compound (C).

[9] The method according to any one of [1] to [8], wherein the reaction temperature is from 140 to 180°C.

[10] The method according to any one of [1] to [9], wherein the reaction time is from 5 to 50 hours.

[11] The method according to any one of [1] to [10], wherein the amount of the compound (B) is from 1 to 120 parts by mass per 100 parts by mass of the compound (A).

[12] A curable composition including the polyalkenylphenol compound (C) according to anyone of [1] to [11].

[13] A cured product of the curable composition according to [12].

[ADVANTAGEOUS EFFECTS OF INVENTION]

According to the present invention, in the production of a polyalkenylphenol compound from a poly 2-alkenyl aromatic ether compound by using a Claisen rearrangement reaction, a polyalkenylphenol compound having a narrow molecular weight distribution and a low viscosity can be obtained in high yield by suppressing a side reaction, such as a polymerization by radical reaction between alkenyl groups. When the polyalkenylphenol compound obtained by the present invention is used as a curing agent in combination with a main agent, such as maleimide, a cured product having high electrical reliability can be obtained. The polyalkenylphenol compound obtained by the present invention is therefore suitable as a raw material of a semiconductor sealing material, etc.

BRIEF DESCRIPTION OF DRAWINGS

[FIG. 1] A $^1$H-NMR spectrum of the product obtained in Synthesis Example 1.
[FIG. 2] A $^1$H-NMR spectrum of the product obtained in Example 1.
[FIG. 3] A $^1$H-NMR spectrum of the product obtained in Example 6.

DESCRIPTION OF EMBODIMENTS

Unless it is specifically indicated to mean a phenol (phenol, $C_6H_5OH$) that is a specific compound, the "phenol compound" as used in the present disclosure means a group of compounds (phenols, phenolics) having a hydroxyl group directly bonded to a carbon atom of an aromatic hydrocarbon group or a property (phenolic) relevant to such a compound.

The present invention is described in detail below. The method for producing a polyalkenylphenol compound of the present invention includes Claisen-rearranging an alkenyl group of a compound (A) having at least two structural units represented by any of formulae (1a), (1b) and (1c) in the presence of at least one compound (B) represented by either formula (3) or (4).

[Compound (A)]

Compound (A) of the present invention is a poly 2-alkenyl aromatic ether compound having at least two structural units represented by any of the following formulae (1a), (1b) and (1c):

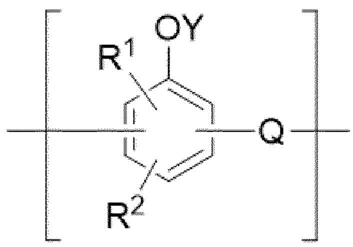
(1a)

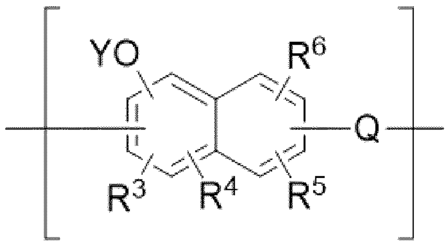
(1b)

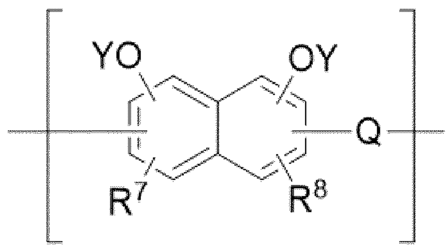
(1c)

In formulae (1a) to (1c), each of $R^1$ to $R^8$ independently represents a hydrogen atom, an alkyl group having a carbon number of 1 to 10, an alkoxy group having a carbon number of 1 to 2, or a hydroxyl group. $R^3$ to $R^8$ may be located on any carbon atom constituting the naphthalene ring. Each Q is independently an alkylene group represented by the formula $-CR^9R^{10}-$, a cycloalkylene group having a carbon number of 5 to 10, a divalent organic group having an aromatic ring, a divalent organic group having an alicyclic fused ring, or a divalent group formed by combination thereof, and each of $R^9$ and $R^{10}$ independently represents a hydrogen atom, an alkyl group having a carbon number of 1 to 5, an alkenyl group having a carbon number of 2 to 6, a cycloalkyl group having a carbon number of 5 to 10, or an aryl group having

a carbon number of 6 to 12. Y is an alkenyl group represented by formula (2):

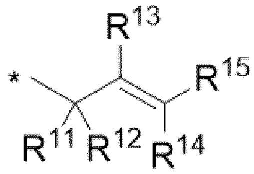
(2)

each of $R^{11}$, $R^{12}$, $R^{13}$, $R^{14}$ and $R^{15}$ independently represents a hydrogen atom, an alkyl group having a carbon number of 1 to 5, a cycloalkyl group having a carbon number of 5 to 10, or an aryl group having a carbon number of 6 to 12, and * in formula (2) represents a bonding site to an oxygen atom. The "each independently" means that a plurality of $R^1$ contained in the compound may be the same as or different from one another. The same holds true for substituents $R^2$ to $R^{15}$, divalent group Q, substituents of compound (B) described blow, etc.

Specific examples of the alkyl group having a carbon number of 1 to 10 include a methyl group, an ethyl group, an n-propyl group, an isopropyl group, an n-butyl group, a sec-butyl group, a tert-butyl group, an n-pentyl group, an n-hexyl group, an octyl group, a nonyl group, and a decyl group.

Specific examples of the alkoxy group having a carbon number of 1 to 2 include a methoxy group and an ethoxy group.

Specific examples of the cycloalkylene group having a carbon number of 5 to 10 include a cyclopentylene group, a cyclohexylene group, a methylcyclohexylene group, and a cycloheptylene group.

The carbon number of the divalent organic group having an aromatic ring is preferably from 6 to 14. Specific examples of the aromatic ring-containing divalent organic group having a carbon number of 6 to 14 include a phenylene group, a methylphenylene group, a naphthylene group, a biphenylene group, a fluorenylene group, an anthracenylene group, a xylylene group, and 4,4-methylenediphenyl group.

The carbon number of the divalent organic group having an alicyclic fused ring is preferably from 6 to 10. Specific examples of the alicyclic fused ring-containing divalent organic group having a carbon number of 6 to 10 include a dicyclopentadienylene group.

Specific examples of the alkyl group having a carbon number of 1 to 5 in $R^9$ and $R^{10}$ include a methyl group, an ethyl group, an n-propyl group, an isopropyl group, an n-butyl group, a sec-butyl group, a tert-butyl group, and an n-pentyl group. Specific examples of the alkenyl group having a carbon number of 2 to 6 include a vinyl group, an allyl group, a butenyl group, a pentenyl group, and a hexenyl group. Specific examples of the cycloalkyl group having a carbon number of 5 to 10 include a cyclopentyl group, a cyclohexyl group, a methylcyclohexyl group, and a cycloheptyl group. Specific examples of the aryl group having a carbon number of 6 to 12 include a phenyl group, a methylphenyl group, an ethylphenyl group, a biphenyl group, and a naphthyl group.

As the divalent group Q, a dicyclopentadienylene group, a phenylene group, a methylphenylene group, and a biphenylene group are preferred from the viewpoint that the mechanical strength is high when used in a curable composition, and on the other hand, $-CH_2$-having a small steric hindrance is preferred in view of the reaction rate.

Specific examples of the alkyl group having a carbon number of 1 to 5, the cycloalkyl group having a carbon number of 5 to 10, and the aryl group having a carbon number of 6 to 12, in $R^{11}$, $R^{12}$, $R^{13}$, $R^{14}$ and $R^{15}$, are the same as those described for $R^9$ and $R^{10}$ in formulae (1a) to (1c). It is preferred that all of $R^{11}$, $R^{12}$, $R^{13}$, $R^{14}$ and $R^{15}$ are a hydrogen atom, that is, the alkenyl group represented by formula (2) is an allyl group.

Compound (A) preferably has at least two structural units represented by either formula (1a) or (1b), more preferably has at least two structural units represented by formula (1a). In some embodiments, compound (A) is composed of one structural unit or two or more structural units selected from the group consisting of formulae (1a), (1b) and (1c). In another embodiment, compound (A) has a structural unit other than the structural unit represented by any of formulae (1a), (1b) and (1c). Compound (A) is preferably composed of one structural unit or two or more structural units selected from the group consisting of formulae (1a) and (1b), more preferably composed of one structural unit or two or more structural units represented by formula (1a).

Compound (A) may be a mixture of compounds differing in the number of structural units. In this embodiment, the average per molecular of the total number of structural units represented by any of formulae (1a), (1b) and (1c) is preferably from 2 to 20, more preferably from 2 to 15. When the average per molecule of the total number of structural units is 2 or more, a cured product using the end product polyalkenylphenol compound as a curing agent has good heat resistance, and when the average is 20 or less, the fluidity during molding is improved.

Specific examples of the raw material polyphenol of compound (A) include known phenol resins, such as phenol novolak resin, cresol novolak resin, triphenylmethane type phenol resin, phenol aralkyl resin, biphenyl aralkyl phenol resin, phenol-dicyclopentadiene copolymer resin, naphthol novolak resin, phenol-naphthol novolak resin and naphthalenediol resin.

The raw material polyphenol is preferably a phenol resin having a number average molecular weight of 500 to 5,000, more preferably from 600 to 3,000. When the number average molecular weight is 500 or more, a cured product using the end product polyalkenylphenol compound as a curing agent has good heat resistance, and when the number average molecular weight is 5,000 or less, the fluidity during molding is improved. In such a phenol resin, a plurality of compounds differing in the molecular weight (number of structural units) are usually mixed. It is not necessary that all compounds contained in the phenol resin are a polyphenol compound having a structural unit corresponding to formulae (1a) to (1c). The phenol resin may include, for example, a binuclear compound in which two phenol skeletons are bonded through methylene. However, the content of such a compound is preferably smaller and is preferably 50 mass% or less, more preferably 30 mass% or less, still more preferably 10 mass% or less, based on the raw material polyphenol.
Compound (A) can be synthesized from the raw material polyphenol by using a known method. For example, compound (A) having at least two structural units represented by any of formulae (1a), (1b) and (1c) can be obtained by reacting the raw material polyphenol and a carboxylic acid 2-alkenyl ester having an alkenyl group represented by formula (2) under basic conditions, for example, in the presence of a transition metal complex catalyst, preferably in the co-presence of a phosphorus compound as a complexing agent.
As the specific reaction method, the method described, for example, in Japanese Unexamined Patent Publication No. 2011-26253, Japanese Translation of PCT International Application No. 10-511721 or Japanese Unexamined Patent Publication No. 2016-028129 may be used. According to the above-described process, a halogen compound derived from the raw material does not get mixed in with the poly 2-alkenyl aromatic ether obtained.

[Compound (B)]

Compound (B) of the present invention is at least one compound represented by either the following formula (3) or (4):

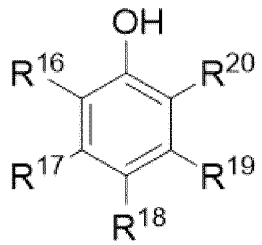
(3)

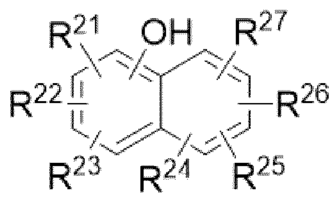
(4)

In formulae (3) and (4), each of $R^{16}$ to $R^{27}$ independently represents a hydrogen atom, an alkyl group having a carbon number of 1 to 10, a cycloalkyl group having a carbon number of 3 to 12, or an aryl group having a carbon number of 6 to 10.
Specific examples of the alkyl group having a carbon number of 1 to 10 in $R^{16}$ to $R^{27}$ include a methyl group, an ethyl group, an n-propyl group, an isopropyl group, an n-butyl group, a sec-butyl group, a tert-butyl group, an n-pentyl group, an n-hexyl group, an octyl group, a nonyl group, and a decyl group. Specific examples of the cycloalkyl group having a carbon number of 3 to 12 include a cyclopropyl group, a cyclobutyl group, a cyclopentyl group, a cyclohexyl group, a methylcyclohexyl group, and a cycloheptyl group. Specific examples of the aryl group having a carbon number of 6 to 10 include a phenyl group, a methylphenyl group, an ethylphenyl group, and a naphthyl group.
The compound represented by formula (3) includes, for example, phenol ($C_6H_5OH$), o-cresol, m-cresol, p-cresol, xylenol, ethylphenol, isopropylphenol, tert-butylphenol, octylphenol, nonylphenol, and phenylphenol. The compound represented by formula (4) includes, for example, 1-naphthol, 2-naphthol, and 2-methyl-1-naphthol. In view of molecular weight and boiling point, a compound represented by formula (3) is preferably used; a compound where each of $R^{16}$ to $R^{20}$ in formula (3) is independently a hydrogen atom or a methyl group is more preferred; phenol, o-cresol, m-cresol, and p-cresol are still more preferred; and phenol is yet still more preferred. A plurality of compounds (B) may also be used in combination.
The boiling point of compound (B) is preferably from 170 to 350°C, more preferably from 175 to 340°C, still

more preferably from 180 to 330°C. When the boiling point is 170°C or more, volatilization of compound (B) during a Claisen rearrangement reaction is effectively prevented, making is possible to accelerate the reaction, and when the boiling point is 350°C or less, compound (B) removal efficiency is increased, so that a high-purity polyalkenylphenol compound (C) can be obtained.

The amount of compound (B) is preferably from 1 to 120 parts by mass, more preferably from 5 to 110 parts by mass, still more preferably from 10 to 100 parts by mass, per 100 parts by mass of compound (A). When the amount of compound (B) is 1 part by mass or more per 100 parts by mass of compound (A), the reaction can be accelerated, and when the amount is 120 parts by mass or less, compound (B) removal efficiency is increased to more enhance the productivity.

[Claisen Rearrangement]

The Claisen rearrangement of compound (A) can be carried out, for example, by mixing compound (A) and compound (B) and heating the obtained mixture. The order and method of addition of compounds (A) and (B) to a reactor are not limited.

The Claisen rearrangement reaction can proceed at a temperature of 140 to 180°C, preferably from 145 to 175°C, more preferably from 150 to 170°C. When the reaction temperature is 140°C or more, the reaction rate is suitable for industrial application, and when the reaction temperature is 180°C or less, a side reaction, such as polymerization, is less likely to occur, and the yield and purity are improved.

The Claisen rearrangement reaction is carried out generally for 5 to 50 hours, preferably for 6 to 40 hours, more preferably for 7 to 30 hours, and the target polyalkenylphenol compound can thereby be obtained. When the reaction time is 5 hours or more, the conversion rate of the Claisen rearrangement reaction is good, and when the reaction time is 50 hours or less, a side reaction, such as polymerization, can be suppressed.

The Claisen rearrangement reaction is carried out in an inert gas atmosphere, such as nitrogen gas and argon, and the target polyalkenylphenol compound can thereby be obtained. When the inside of the reaction system is placed in an inert gas atmosphere, a side reaction, such as coloring of the target product due to oxidation or production of an insoluble component due to polymerization, etc., can be suppressed.

In the Claisen rearrangement reaction, other additives may also be added as long as the progress of the reaction is not excessively inhibited.

In the Claisen rearrangement reaction, the rate of molecular weight increase defined by the following formula is preferably from 0 to 70%, more preferably from 0 to 60%, still more preferably from 0 to 50%. When the rate of molecular weight increase is 70% or less, the viscosity of the product after the reaction is low and therefore, the productivity is improved.

$$\text{Rate of molecular weight increase [\%]} = (m_C / m_A - 1) \times 100$$

$m_A$ represents the weight average molecular weight of compound (A) and $m_C$ represents the weight average molecular weight of polyalkenylphenol compound (C).

After the Claisen rearrangement reaction is carried out, compound (B) can be removed, if desired. Removal of compound (B) can be carried out by a known distillation method, such as normal pressure distillation, reduced pressure distillation and molecular distillation, by using a known distillation apparatus, such as continuous distillation apparatus, batch distillation apparatus and thin-film distillation apparatus. Compound (B) is preferably removed by distillation under reduced pressure. When the distillation is carried out under reduced pressure, a side reaction, such as oxidation and polymerization of the produced polyalkenylphenol compound (C), can be suppressed.

[Polyalkenylphenol Compound (C)]

Polyalkenylphenol compound (C) obtained by the present invention is a poly 2-alkenylphenol compound having at least two structural units represented by any of the following formulae (5a), (5b) and (5c):

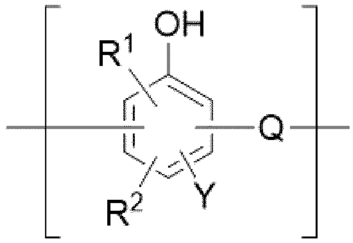

(5a)

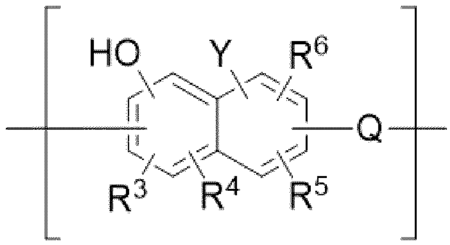

(5b)

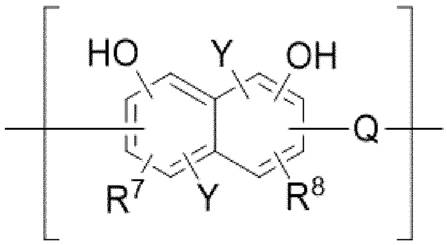

(5c)

In formulae (5a) to (5c), $R^1$ to $R^8$ and Q are as described for formula (1a) to (1c), and Y is as described for formula (2).

[Use Method of Polyalkenylphenol Compound (C)]

Polyalkenylphenol compound (C) obtained by the present invention can be used as a component of a curable composition. For example, a thermosetting composition having low viscosity and excellent moldability or coatability can be obtained by combining polyalkenylphenol compound (C) with an aromatic bismaleimide compound, and a polymerization initiator as a curing accelerator. Polyalkenylphenol compound (C) can also be used in a radiation-sensitive composition. A composition containing polyalkenylphenol compound (C) and a cured product thereof can be used, for example, in applications, such as a semiconductor sealing material, a prepreg, an interlayer insulating resin, a solder resist and a die attach.

EXAMPLES

The present invention is specifically described below based on Examples and Comparative Examples, but the present invention is not limited to these Examples.

[Analysis Method]

•Yield

Denoting M1 [g] as the amount of the poly 2-alkenyl aromatic ether compound (in the following Example, a polyallyl aromatic ether compound) charged before the Claisen rearrangement reaction and M2 [g] as the amount of the polyalkenylphenol compound taken out after the Claisen rearrangement reaction, the yield was determined according to the following formula:

$$\text{Yield [\%]} = M2 / M1 \times 100$$

•Conversion Rate in Claisen Rearrangement Reaction

Assuming the integrated value of δ4.6 to 4.4 ppm (signals based on hydrogen atom on the carbon atom bonded to oxygen atom in the alkenyl group Y of formulae (1a) to (1c)) in the $^1$H-NMR spectrum of the poly 2-alkenyl aromatic ether compound before the Claisen rearrangement reaction is 100, the conversion rate was calculated from the decrease rate of the integrated value of δ4.6 to 4.4 ppm in the $^1$H-NMR spectrum of the reaction product after the Claisen rearrangement reaction. The apparatus and the measurement conditions used are as follows.
Apparatus name: JEOL AL400 (manufactured by JEOL Ltd.)
Solvent: deuterated chloroform
Temperature: 27°C

•Measurement of Molecular Weight by GPC

The measurement conditions of GPC are as follows.
Apparatus name: JASCO LC-2000 plus (manufactured by JASCO Corp.)
Column: Shodex (registered trademark) LF-804 (manufactured by Showa Denko K.K.)
Mobile phase: tetrahydrofuran
Flow velocity: 1.0 mL/min
Detector: JASCO RI-2031 plus (manufactured by JASCO Corp.)
Temperature: 40°C
Under the measurement conditions above, the number average molecular weight Mn and the weight average molecular weight Mw were calculated using a calibration curve created with a polystyrene standard substance. Denoting $m_A$ as the weight average molecular weight of the polyallyl aromatic ether compound before the Claisen rearrangement reaction and $m_C$ as the weight average molecular weight of the polyalkenylphenol compound after the Claisen rearrangement reaction, the rate of molecular weight increase was determined according to the following formula:

$$\text{Rate of molecular weight increase } [\%] = (m_C / m_A - 1) \times 100$$

•Melt Viscosity

0.5 g of sample was placed in a rheometer (rotary viscometer) and measured using a cone and plate (CP-15). The measurement conditions are as follows.
Apparatus name: Viscoelasticity measuring apparatus Bohlin C-VOR (manufactured by Malvern Instruments)
Temperature: 100°C

[Synthesis Example 1] Production of Polyallyl Ether Resin A-1

A 1,000 mL three-neck flask was charged with a solution obtained by dissolving 201 g (1.45 mol) of potassium carbonate (produced by Nippon Soda Co., Ltd.) in 150 g of pure water, and 150.0 g (hydroxyl group: 1.4 mol) of a 1:1 mixture of phenol novolak resin SHONOL (registered trademark) BRG-556 (produced by Showa Denko K.K., number average molecular weight: 600, weight average molecular weight: 850) and phenol novolak resin SHONOL (registered trademark) BRG-558 (produced by Showa Denko K.K., number average molecular weight: 1,050, weight average molecular weight: 1,850), and the reactor was purged with nitrogen gas and heated at 85°C. Under a nitrogen gas flow, 204 g (2.04 mol) of allyl acetate (produced by Showa Denko K.K.), 3.82 g (14.6 mmol) of triphenylphosphine (produced by Hokko Chemical Industry Co., Ltd.), and 0.62 g (0.291 mmol) of a 50 mass% hydrous 5 mass%-Pd/C-STD type (produced by N.E. Chemcat Corporation) were put in the flask, and the temperature was raised to 105°C in a nitrogen gas atmosphere. After the reaction for 4 hours, 29 g (0.291 mol) of allyl acetate was additionally added, and heating was continued for 10 hours. Thereafter, the stirring was stopped, and the system was left standing still and thereby separated into two layers of an organic layer and an aqueous layer. Pure water (200 g) was added until the precipitated salt (potassium acetate salt) was dissolved, and 200 g of toluene was then added. After keeping a temperature of 80°C or higher and confirming that white precipitate (potassium acetate) was not present, Pd/C was recovered by filtration (using a 1 micrometer membrane filter (KST-142-JA manufactured by ADVANTEC Co., Ltd.) under pressure (0.3 MPa)). The filter cate was washed with 100 g of toluene and at the same time, the aqueous layer was separated. The filter cake washing toluene was combined with the organic layer and washed three times with 200 g of pure water and after third washing, the pH of the aqueous layer separated was confirmed to be 7.0. The separated organic layer was added with 4.5 g of activated carbon CN1 (produced by Norit Japan K.K.), heated at 60°C and stirred for 1 hour at 300 rpm by

means of a magnetic stirrer. Thereafter, the activated carbon was removed by filtration (using a 1 micrometer membrane filter (KST-142-JA manufactured by ADVANTEC Co., Ltd.) under pressure (0.3 MPa)), and the residue was then concentrated (removal of toluene and excess allyl acetate) under reduced pressure to obtain a brown oily product A-1. Identification by $^1$H-NMR spectrum revealed that A-1 is a phenol novolak-type polyallyl ether resin where in formula (1a), $R^1 = R^2 = R^9 = R^{10}$ = hydrogen atom and Q = $-CR^9R^{10}-$ and in formula (2), $R^{11}$ to $R^{15}$ = hydrogen atom (Fig. 1). The number average molecular weight was 600, the weight average molecular weight was 1,200, and the yield was 96%.

[Synthesis Example 2] Production of Polyallyl Ether Resin A-2

A-2 was obtained by the same operation as in Synthesis Example 1 except that 150.0 g of triphenylmethane-type phenol resin SHONOL (registered trademark) TRI-002 (produced by Showa Denko K.K., number average molecular weight: 500, weight average molecular weight: 600) was used in place of 150.0 g of the 1:1 mixture of phenol novolak resins SHONOL (registered trademark) BRG-556 and BRG-558. Identification by $^1$H-NMR spectrum revealed that A-2 is a polyallyl ether resin of triphenylmethane-type phenol, where in formula (1a), $R^1 = R^2 = R^9$ = hydrogen atom, $R^{10}$ = $C_6H_4OH$, and Q = $-CR^9R^{10}-$ and in formula (2), $R^{11}$ to $R^{15}$ = hydrogen atom. The number average molecular weight was 500, the weight average molecular weight was 600, and the yield was 95%.

[Synthesis Example 3] Production of Polyallyl Ether Resin A-3

A-3 was obtained by the same operation as in Synthesis Example 1 except that 149.5 g of naphthalenediol resin SN-395 (produced by Nippon Steel Chemical Co., Ltd., number average molecular weight: 550, weight average molecular weight: 1,200) was used in place of 150.0 g of the 1:1 mixture of phenol novolak resins SHONOL (registered trademark) BRG-556 and BRG-558. Identification by $^1$H-NMR spectrum revealed that A-3 is a naphthalenediol-type polyallyl ether resin where in formula (1c), $R^7 = R^8$ = hydrogen atom and Q = $-CH_2-C_6H_4-CH_2-$ and in formula (2), $R^{11}$ to $R^{15}$ = hydrogen atom. The number average molecular weight was 600, the weight average molecular weight was 1,200, and the yield was 93%.

[Example 1]

200 g of phenol novolak-type polyallyl ether resin A-1 obtained in Synthesis Example 1 and 200 g of phenol ($C_6H_5OH$, produced by Junsei Chemical Co., Ltd., boiling point: 182°C) were put in a 1,000 mL separable flask. A nitrogen gas was blown into the reactor, the temperature was raised to 170°C while stirring at 300 rpm with a mechanical stirrer, and immediately, a Claisen rearrangement reaction was allowed to proceed for 7 hours in a nitrogen gas atmosphere. Thereafter, phenol was removed at 160°C under reduced pressure to obtain a red-brown reaction product. Identification by $^1$H-NMR spectrum revealed that the reaction product is a phenol novolak-type polyallylphenol resin in which in formula (5a), $R^1 = R^2 = R^9 = R^{10}$ = hydrogen atom and Q = $-CR^9R^{10}-$ and in formula (2), $R^{11}$ to $R^{15}$ = hydrogen atom (Fig. 2). The structural unit of the obtained phenol novolak-type polyallylphenol resin is shown in formula (5a1), and other evaluation results are shown in Table 1.

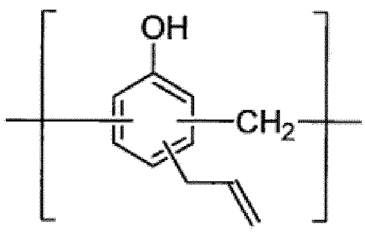
(5a1)

[Example 2]

The reaction was carried out in the same manner as in Example 1 except that the amount of phenol added in Example 1 was changed to 66 g, and the reaction product was confirmed by $^1$H-NMR. As a result, the target polyallylphenol resin was obtained in a reaction time of 17 hours. The evaluation results are shown in Table 1.

[Example 3]

The reaction was carried out in the same manner as in Example 1 except that the amount of phenol added in Example 1 was changed to 20 g, and the reaction product was confirmed by $^1$H-NMR. As a result, the target polyallylphenol

resin was obtained in a reaction time of 21 hours. The evaluation results are shown in Table 1.

[Example 4]

The reaction was carried out in the same manner as in Example 1 except that the reaction temperature in Example 1 was changed to 150°C, and the reaction product was confirmed by $^1$H-NMR. As a result, the target polyallylphenol resin was obtained in a reaction time of 30 hours. The evaluation results are shown in Table 1.

[Example 5]

The same operation as in Example 1 was carried out except that polyallyl ether resin A-2 obtained in Synthesis Example 2 was used in place of polyallyl ether resin A-1. The reaction product was confirmed by $^1$H-NMR. As a result, it was found that a solid triphenylmethane-type polyallylphenol resin where in formula (5a), $R^1 = R^2 = R^9$ = hydrogen atom, $R^{10} = C_6H_4OH$, and Q = $-CR^9R^{10}-$ and in formula (2), $R1^1$ to $R^{15}$ = hydrogen atom, was obtained. The structural unit of the obtained triphenylmethane-type polyallylphenol resin is shown in formula (5a2), and evaluation results are shown in Table 1.

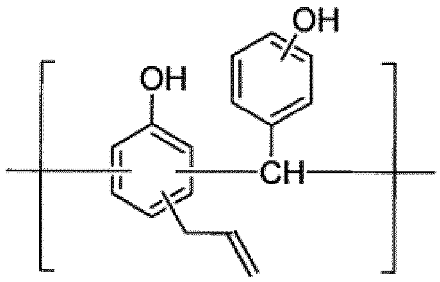
(5a2)

[Example 6]

The same operation as in Example 1 was carried out except that polyallyl ether resin A-3 obtained in Synthesis Example 3 was used in place of polyallyl ether resin A-1. The reaction product was confirmed by $^1$H-NMR. As a result, it was found that a solid polyallylnaphthalenediol resin where in formula (5c), $R^7 = R^8$ = hydrogen atom and Q = $-CH_2-C_6H_4-CH_2-$ and in formula (2), $R^{11}$ to $R^{15}$ = hydrogen atom, was obtained (Fig. 3). The structural unit of the obtained polyallylnaphthalenediol resin is shown in formula (5c1), and evaluation results are shown in Table 1.

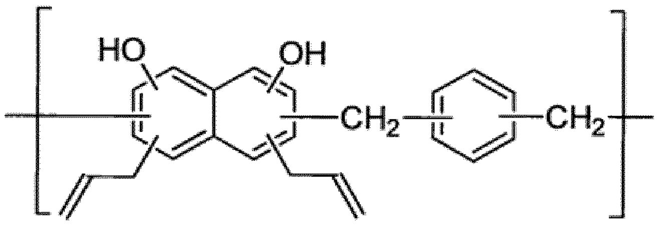
(5c1)

[Example 7]

The same operation as in Example 1 was carried out except that p-cresol ($C_7H_7OH$, produced by Junsei Chemical Co., Ltd., boiling point: 202°C) was used in place of phenol in Example 1. The reaction product was confirmed by $^1$H-NMR, as a result, the target polyallylphenol resin was obtained in a reaction time of 8 hours. The evaluation results are shown in Table 1.

[Comparative Example 1]

The reaction was carried out in the same manner as in Example 1 except that phenol was not added in Example 1. The reaction product was confirmed by $^1$H-NMR. As a result, the target polyallylphenol resin was obtained in a reaction time of 27 hours. The evaluation results are shown in Table 1.

[Comparative Example 2]

The reaction was carried out in the same manner as in Example 5 except that phenol was not added in Example 5. The reaction product was confirmed by $^1$H-NMR. As a result, the target polyallylphenol resin was obtained in a reaction time of 27 hours. The evaluation results are shown in Table 1.

[Comparative Example 3]

The reaction was carried out in the same manner as in Example 6 except that phenol was not added in Example 6. The reaction product was confirmed by $^1$H-NMR. As a result, the target polyallylphenol resin was obtained in a reaction time of 27 hours. The evaluation results are shown in Table 1.

Table 1

| | Raw Material | | Claisen Rearrangement Reaction Conditions | | | | | Product | | | | |
|---|---|---|---|---|---|---|---|---|---|---|---|---|
| | Compound (A) | Compound (B) | (B) per 100 parts by mass of (A) (parts by mass) | Reaction Temperature (°C) | Reaction Time (hours) | Conversion Rate (%) | Yield (%) | Mn | Mw | Molecular Weight Distribution Mw/Mn | Rate of Molecular Weight Increase (%) | Melt Viscosity 1) mPa·s |
| Example 1 | Resin A-1 | phenol | 100 | 170 | 7 | 100 | 96 | 850 | 1550 | 1.8 | 29 | 400 |
| Example 2 | Resin A-1 | phenol | 33 | 170 | 17 | 100 | 95 | 850 | 1550 | 1.8 | 29 | 400 |
| Example 3 | Resin A-1 | phenol | 10 | 170 | 21 | 100 | 95 | 850 | 1550 | 1.8 | 29 | 400 |
| Example 4 | Resin A-1 | phenol | 100 | 150 | 30 | 100 | 95 | 850 | 1550 | 1.8 | 29 | 400 |
| Example 5 | Resin A-2 | phenol | 100 | 170 | 7 | 100 | 94 | 500 | 600 | 1.2 | 0 | 1500 |
| Example 6 | Resin A-3 | phenol | 100 | 170 | 7 | 100 | 93 | 800 | 1750 | 2.2 | 46 | * |
| Example 7 | Resin A-1 | p-cresol | 100 | 170 | 8 | 100 | 95 | 850 | 1550 | 1.8 | 29 | 400 |
| Comparative Example 1 | Resin A-1 | - | 0 | 170 | 27 | 100 | 96 | 1050 | 3000 | 2.9 | 150 | 1200 |
| Comparative Example 2 | Resin A-2 | - | 0 | 170 | 27 | 100 | 90 | 900 | 1700 | 1.9 | 183 | * |
| Comparative Example 3 | Resin A-3 | - | 0 | 170 | 27 | 100 | 90 | 1050 | 3500 | 3.3 | 192 | * |

1) * indicates that measurement was not carried out.

It is understood from Table 1 that the target polyalkenyl (allyl) phenol compound having a 2-alkenyl (allyl) group is obtained in high yield and high purity in Examples, as compared with Comparative Examples.

[Production of Curable Composition]

The polyalkenylphenol compound (phenol novolak-type polyallyl phenol resin) obtained in Example 1 and the following raw materials were mixed to obtain a curable composition.

•Aromatic bismaleimide compound:

BMI-4000 (2,2'-bis[4-(4-maleimidophenyloxy)phenyl]propane, melting point: 165°C, Daiwakasei Industry Co,. Ltd.)

•Curing accelerator:

PERCUMYL (registered trademark) D (dicumyl peroxide, NOF Corporation)

•Silica filler:

MSR2212 (spherical silica, average particle diameter: 25.5 μm, Tatsumori Ltd., treated using 0.5 mass% of silane coupling agent KBM-403 (Shin-Etsu Chemical Co., Ltd.))

100 Parts by mass of BMI-4000 was added to a reactor and stirred under heating at 170°C. When BMI-4000 all was melted to provide a transparent liquid material, the temperature was lowered to 150°C. 100 Parts by mass of the polyalkenylphenol compound of Example 1 having been heated at 80°C and melted was added to the reactor, and two compounds were mixed with stirring under heating at 150°C for 10 minutes. The obtained mixture and respective components shown in Table 2 were blended in the ratio shown in the Table and melt-kneaded (with a twin roll (roll diameter: 8 inches) manufactured by Toyo Seiki Co., Ltd., 110°C, 10 minutes). Subsequently, the kneaded product was allowed to cool at room temperature (25°C) for 1 hour, thereby solidified and then pulverized using a mill mixer (manufactured by Osaka Chemical Co., Ltd., Model WB-1, 25°C, 30 seconds) to obtain the target curable composition in a powdered state.

[Method for Evaluating Properties of Cured Product]

•Glass transition temperature (Tg)

The powdered curable composition was molded by a transfer molding machine (manufactured by Matsuda Seisakusho K.K.) under the conditions of a mold temperature of 180°C, a holding pressure of 100 kg/cm$^2$, and a holding time of 3 minutes to manufacture a test piece for glass transition temperature measurement. The test piece was heated at 230°C for 6 hours, then cured and measured by thermomechanical analysis (TMA). A test piece of 5 mm × 5 mm × 5 mm was measured by using a thermomechanical analyzer TMA/SS6100 manufactured by SII NanoTechnology Inc. under the conditions of a temperature range of 30 to 300°C, a temperature rise rate of 5°C/min and a load of 20.0 mN, and the temperature at the inflection point of linear expansion coefficient is defined as Tg. The results are shown in Table 2.

•Bending strength and bending modulus

The powdered curable composition was molded by a transfer molding machine (manufactured by Matsuda Seisakusho K.K.) under the conditions of a mold temperature of 180°C, a holding pressure of 100 kg/cm$^2$, and a holding time of 3 minutes to manufacture a test piece for three-point bending test. The test piece was heated at 230°C for 6 hours, then cured and measured by using a Tensilon tester (model: MSAT0002RTF/RTG) manufactured by A&D Company Limited. The shape of the test piece was 750 mm (length) × 10 mm (width) × 3 mm (thickness). A three-point bending test was carried out 5 times at room temperature and a test speed of 2 mm/min according to JIS K 7171, and the average values thereof are defined as the bending strength and the bending modulus. The results are shown in Table 2.

•Dielectric constant and dielectric loss tangent

These were determined by complex permittivity measurement using a cavity resonator perturbation method. The powdered curable composition was molded by a transfer molding machine (manufactured by Matsuda Seisakusho K.K.) under the conditions of a mold temperature of 180°C, a holding pressure of 100 kg/cm$^2$, and a holding time of 3 minutes to manufacture a test piece for dielectric constant and dielectric loss tangent measurements. The test piece

was heated at 230°C for 6 hours and then cured, and a test piece of 1.5 mm × 1.5 mm × 70.0 mm was measured under the conditions of room temperature and a measurement frequency of 5 GHz by using 8753ES S parameter-vector-network-analyzer produced by Keysight Technologies Japan G.K., a cavity resonator perturbation program CPMA-V3, and a cavity resonator CP19 for 5 GHz to determine the values of dielectric constant and dielectric loss tangent. The results are shown in Table 2.

Table 2

| | | |
|---|---|---|
| Blending (parts by mass) | Polyalkenylphenol compound of Example 1 | 100 |
| | Aromatic bismaleimide compound | 100 |
| | Curing accelerator | 0.65 |
| | Silica filler | 800 |
| Evaluation results | Glass transition temperature (°C) | 281 |
| | Bending modulus (GPa) | 17.1 |
| | Bending strength (MPa) | 136.3 |
| | Dielectric constant | 3.4 |
| | Dielectric loss tangent | 0.0053 |

INDUSTRIAL APPLICABILITY

Combining the polyalkenylphenol compound having a narrow molecular weight distribution and a low viscosity produced by the present invention as a curing agent with a main agent, such as maleimide, can achieve good moldability and provide a cured product having high electrical reliability. The polyalkenylphenol compound having a narrow molecular weight distribution and a low viscosity produced by the present invention can be suitably used as a raw material, etc., of a semiconductor sealing material.

## Claims

1. A method for producing a polyalkenylphenol compound (C), comprising Claisen-rearranging an alkenyl group of a compound (A) having at least two structural units represented by any of the following formulae (1a), (1b) and (1c):

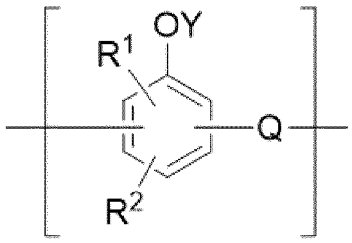

(1a)

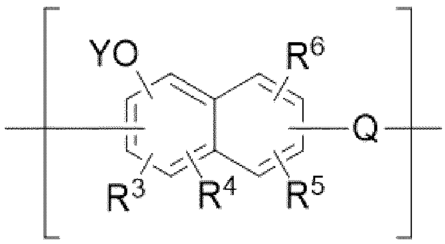

(1b)

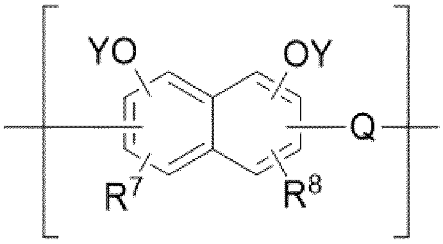

(1c)

,

wherein, in formulae (1a) to (1c), each of $R^1$ to $R^8$ independently represents a hydrogen atom, an alkyl group having a carbon number of 1 to 10, an alkoxy group having a carbon number of 1 to 2, or a hydroxyl group, $R^3$ to $R^8$ may be located on any carbon atom constituting the naphthalene ring, each Q is independently an alkylene group represented by the formula - $CR^9R^{10}$-, a cycloalkylene group having a carbon number of 5 to 10, a divalent organic group having an aromatic ring, a divalent organic group having an alicyclic fused ring, or a divalent group formed by combination thereof, each of $R^9$ and $R^{10}$ independently represents a hydrogen atom, an alkyl group having a carbon number of 1 to 5, an alkenyl group having a carbon number of 2 to 6, a cycloalkyl group having a carbon number of 5 to 10, or an aryl group having a carbon number of 6 to 12, Y is an alkenyl group represented by the following formula (2):

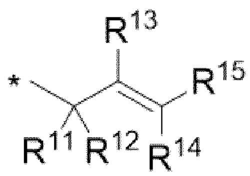

(2)

each of $R^{11}$, $R^{12}$, $R^{13}$, $R^{14}$ and $R^{15}$ independently represents a hydrogen atom, an alkyl group having a carbon number of 1 to 5, a cycloalkyl group having a carbon number of 5 to 10, or an aryl group having a carbon number of 6 to 12, and * in formula (2) represents a bonding site to an oxygen atom,
in the presence of at least one compound (B) represented by either the following formula (3) or (4):

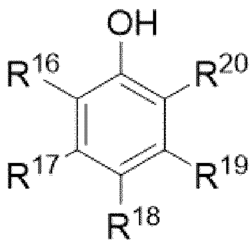

(3)

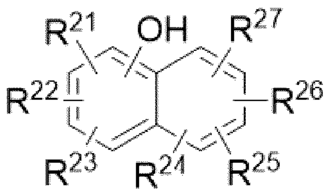

(4)

wherein, in formulae (3) and (4), each of $R^{16}$ to $R^{27}$ independently represents a hydrogen atom, an alkyl group having a carbon number of 1 to 10, a cycloalkyl group having a carbon number of 3 to 12, or an aryl group having a carbon number of 6 to 10.

2. The method according to claim 1, wherein the compound (B) is a compound represented by formula (3).

3. The method according to claim 1 or 2, wherein the compound (B) is a compound represented by formula (3) and each of $R^{16}$ to $R^{20}$ is independently a hydrogen atom or a methyl group.

**4.** The method according to any one of claims 1 to 3, wherein the compound (B) is at least one compound selected from the group consisting of phenol, o-cresol, m-cresol, and p-cresol.

**5.** The method according to any one of claims 1 to 4, wherein the compound (A) has at least two structural units represented by either formula (1a) or (1b).

**6.** The method according to any one of claims 1 to 5, wherein the compound (A) has at least two structural units represented by formula (1a).

**7.** The method according to any one of claims 1 to 6, wherein in the compound (A), the average per molecule of the total number of structural units represented by any of formulae (1a), (1b) and (1c) is from 2 to 20.

**8.** The method according to any one of claims 1 to 7, wherein the rate of molecular weight increase defined by the following formula is from 0 to 70%:

$$\text{Rate of molecular weight increase [\%]} = (m_C / m_A - 1) \times 100,$$

wherein $m_A$ represents the weight average molecular weight of the compound (A) and $m_C$ represents the weight average molecular weight of the polyalkenylphenol compound (C).

**9.** The method according to any one of claims 1 to 8, wherein the reaction temperature is from 140 to 180°C.

**10.** The method according to any one of claims 1 to 9, wherein the reaction time is from 5 to 50 hours.

**11.** The method according to any one of claims 1 to 10, wherein the amount of the compound (B) is from 1 to 120 parts by mass per 100 parts by mass of the compound (A).

**12.** A curable composition comprising the polyalkenylphenol compound (C) according to any one of claims 1 to 11.

**13.** A cured product of the curable composition according to claim 12.

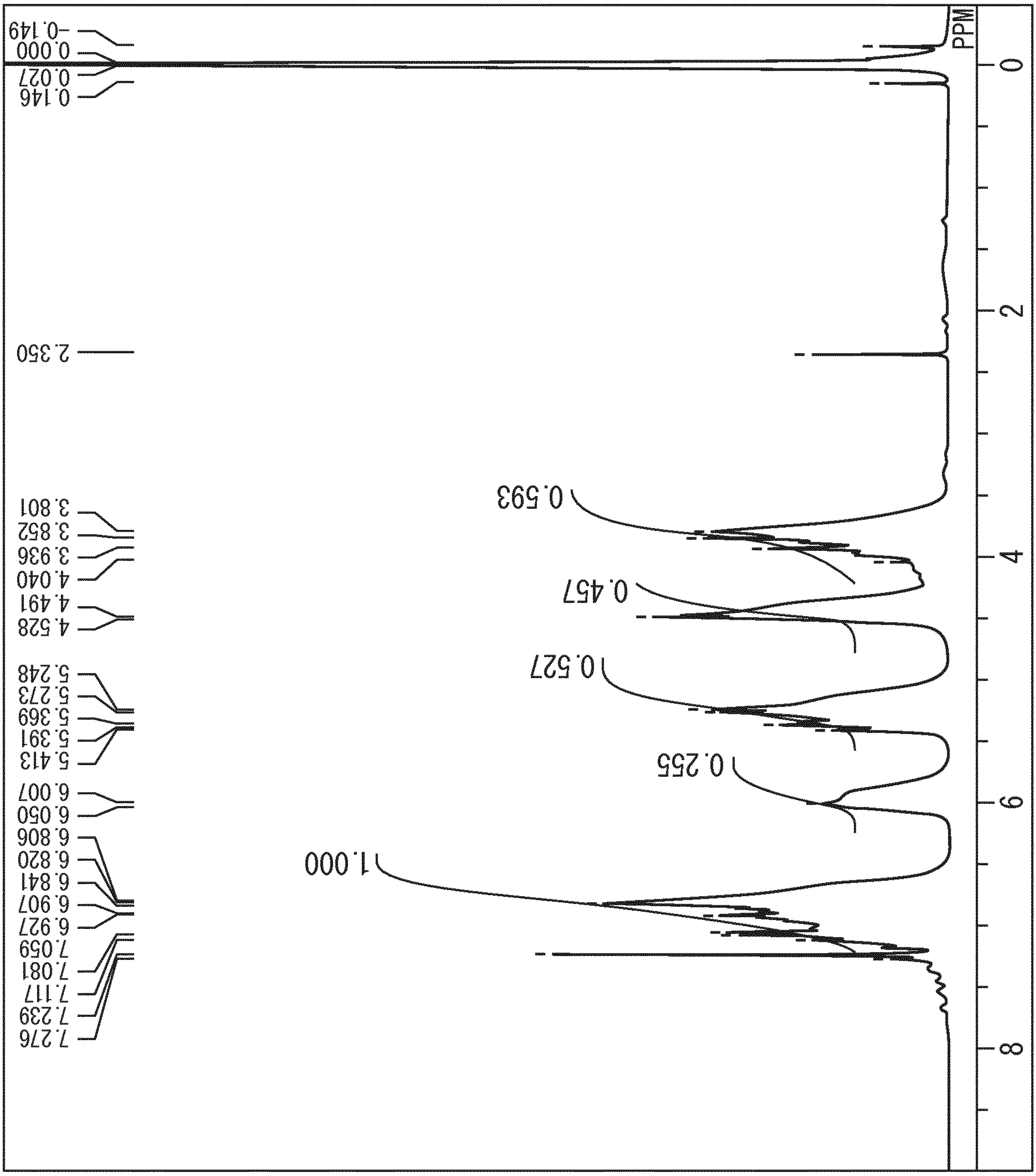

-0.149
0.000
0.027
0.146
2.350
3.801
3.852
3.936
4.040
4.491
4.528
5.248
5.273
5.369
5.391
5.413
6.007
6.050
6.806
6.820
6.841
6.907
6.927
7.059
7.081
7.117
7.239
7.276
0.593
0.457
0.527
0.255
1.000
PPM
0
2
4
6
8


FIG. 1

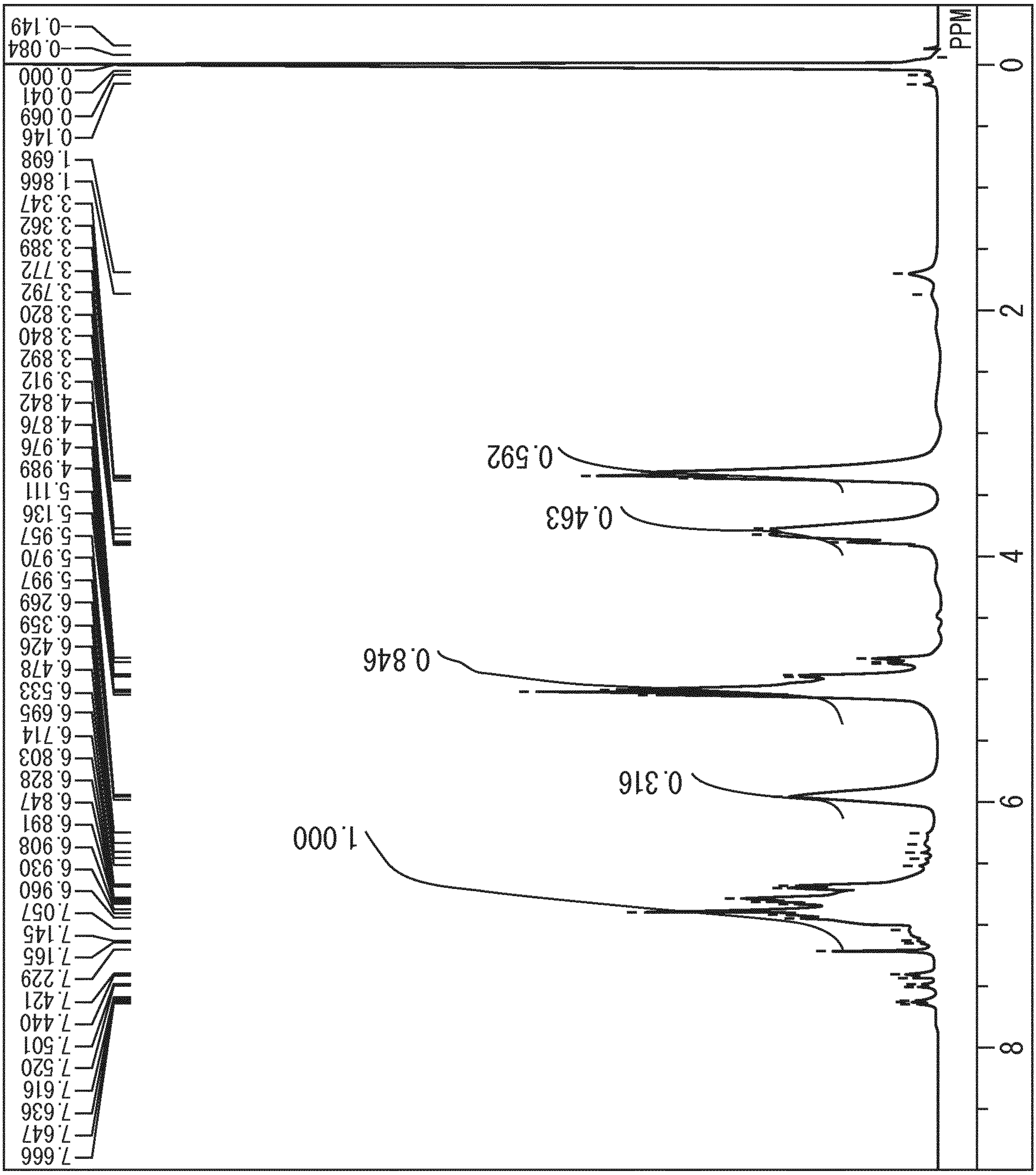
-0.149
-0.084
0.000
0.041
0.069
0.146
1.698
1.866
3.347
3.362
3.389
3.772
3.792
3.820
3.840
3.892
3.912
4.842
4.876
4.976
4.989
5.111
5.136
5.957
5.970
5.997
6.269
6.359
6.426
6.478
6.533
6.695
6.714
6.803
6.828
6.847
6.891
6.908
6.930
6.960
7.057
7.145
7.165
7.229
7.421
7.440
7.501
7.520
7.616
7.636
7.647
7.666
0.592
0.463
0.846
0.316
1.000
PPM
0
2
4
6
8

FIG. 2

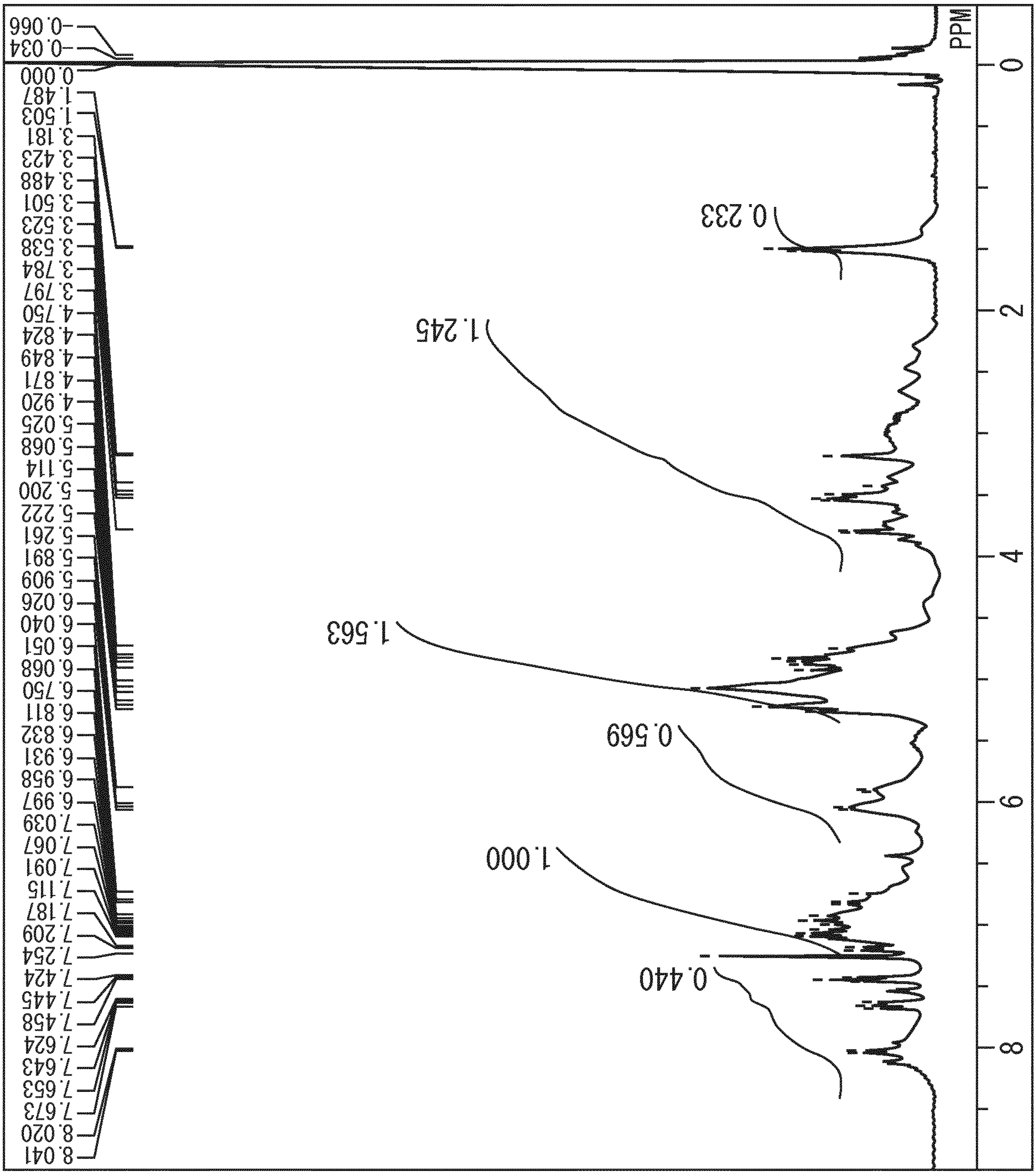
-0.066
-0.034
0.000
1.487
1.503
3.181
3.423
3.488
3.501
3.523
3.538
3.784
3.797
4.750
4.824
4.849
4.871
4.920
5.025
5.068
5.114
5.200
5.222
5.261
5.891
5.909
6.026
6.040
6.051
6.068
6.750
6.811
6.832
6.931
6.958
6.997
7.039
7.067
7.091
7.115
7.187
7.209
7.254
7.424
7.445
7.458
7.624
7.643
7.653
7.673
8.020
8.041
0.233
1.245
1.563
0.569
1.000
0.440
PPM
0
2
4
6
8

FIG. 3

## INTERNATIONAL SEARCH REPORT

International application No.
PCT/JP2017/028997

A. CLASSIFICATION OF SUBJECT MATTER
*C08G8/30*(2006.01)i, *C07C39/04*(2006.01)i, *C07C39/07*(2006.01)i

According to International Patent Classification (IPC) or to both national classification and IPC

B. FIELDS SEARCHED

Minimum documentation searched (classification system followed by classification symbols)
C08G8/30, C07C39/04, C07C39/07

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/WPIDS(STN)

C. DOCUMENTS CONSIDERED TO BE RELEVANT

| Category* | Citation of document, with indication, where appropriate, of the relevant passages | Relevant to claim No. |
|---|---|---|
| A | WO 2016/010124 A1 (Showa Denko Kabushiki Kaisha), 21 January 2016 (21.01.2016), entire text & KR 10-2016-0135334 A & CN 106462064 A | 1-13 |
| A | JP 2012-107145 A (Nitto Denko Corp.), 07 June 2012 (07.06.2012), entire text (Family: none) | 1-13 |
| A | JP 2012-102282 A (Nitto Denko Corp.), 31 May 2012 (31.05.2012), entire text (Family: none) | 1-13 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 October 2017 (26.10.17) | 07 November 2017 (07.11.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No. PCT/JP2017/028997

C (Continuation). DOCUMENTS CONSIDERED TO BE RELEVANT

| Category* | Citation of document, with indication, where appropriate, of the relevant passages | Relevant to claim No. |
|---|---|---|
| A | JP 62-29543 A (Mitsui Toatsu Chemicals, Inc.), 07 February 1987 (07.02.1987), entire text (Family: none) | 1-13 |
| A | MINAMI, K. et al., DIASTEREOCONTROL VIA THE PHENOL- AND PALLADIUM(II)-CATALYZED CLAISEN REARRANGEMENT WITH CYCLIC ENOL ETHERS, TETRAHEDRON LETTERS, 1987, 28, 47, 5879-5882 | 1-13 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004137200 A
- JP 2003104923 A
- JP 2011026253 A
- JP 10511721 A
- JP 2016028129 A